# EUROPEAN PATENT SPECIFICATION

(11) **EP 3 492 026 B1**
(45) Date of publication and mention of the grant of the patent: **22.07.2026**
(21) Application number: 18189476.7
(22) Date of filing: 17.08.2018
(51) Int. Cl.: A61B 17/16

(54) **SURGICAL REAMER FOR REMOVING BONE**
CHIRURGISCHER REAMER ZUR ENTFERNUNG VON KNOCHEN
ALÉSOIR CHIRURGICAL PERMETTANT DE RETIRER DES OS

(30) Priority: 18.08.2017 GB 201713322
(43) Date of publication of application: 05.06.2019
(73) Proprietor: Hoogland Spine Products GmbH, 85774 Unterföhring (DE)
(72) Inventor: HOOGLAND, Jaap Johannes, 80799 München (DE)
(74) Representative: Hoffmann Eitle

(56) References cited:
- WO-A1-2014/036178
- GB-A- 2 430 396
- US-A1- 2007 010 822
- US-A1- 2016 296 344

## Description

### Field of the Invention

The present invention relates to a surgical reamer for removing bone. In particular, the present invention relates to a surgical reamer for use, for example, in the field of spinal surgery. Further, the present invention relates to a set of reamers for removing bone.

### Technical Background

In the field of spinal surgery, there is a need to expose the internal spinal structures by removing parts of the facet joints and the facet joint capsule by a posterior or posterolateral approach. The removal of the bone structure to access the spinal canal is a very delicate and sensitive procedure. It has to be avoided by any means that the nerve sack (dura) or one of the branching nerve routes is injured or severed, since such an injury is very dangerous and the patient may not recover.

This is difficult to achieve with conventional reamers because they remove bone material by a rotational movement which easily leads to tissue injuries in the area surrounding the section to be cut inside the patient leading to a prolonged recovery process.

US 2009/0259227 A1 discloses a reamer for operating implants, which can apply an implant operation to a patient, who is short of bone quantity in the jaw. In particular, the reamer includes a protruding face formed on the top surface thereof in such a manner as to upwardly upheave a partial surface including an outer circumference of the cutting part of the entire top surface of the cutting parts to form a stepped jaw. Additionally, the surgical reamer has a transportation path being disposed on the circumferential outer side of a shaft of the reamer for guiding removed material to a proximal end of the shaft. However, this surgical reamer does not show a protective surface in order to protect the tissue, nerves, etc. surrounding the cutting area in the patient's body and furthermore shows a rotational movement, so that the reamer is not able to avoid injuries at the surrounding area of the cutting area inside the patient's body during the operation. This leads to a prolonged healing process.

US 5,931,841 discloses a reamer which is generally symmetrical about its longitudinal axis and is provided with at least one substantially longitudinal flute defining a sharp cutting edge which extends generally longitudinally along substantially the entire cutting portion of the tool. The reamer has a shaft which allows to be rotated about its longitudinal axis with the aid of a handle or a motor. The reamer can also include a plurality of flutes which are helically arranged and have a plurality of cutting edges. However, since the cutting flute does not extend along part of the circumference of the shaft it is not possible to achieve a precise cutting of the bone material inside the patient. Further, as the reamer is rotated, a protection of tissue, nerves, etc. surrounding the cutting area cannot be achieved and a protective surface is not attainable.

DE 198 50 980 A1 discloses a reamer for preparing a bone cavity for receiving an implant. The reamer has two straight, double-edged, longitudinally adjacent bits with a longitudinal axis sloping towards each other. A number of blades run parallel with the longitudinal axis and the peripheral surface between the two blades is concave. The blades extend of the entire proximal part and over the adjacent part of the distal part. However, the cutting section is not arranged at a distal tip of the reamer and therefore has to be inserted deeply into the patient's body and arranging the cutting section at the right spot is difficult to achieve.

Other related art may be found in WO 2014/036178 A1, which is the closest prior art to the subject-matter of claim 1 and is directed to a dilator tube comprising a central longitudinal axis, an inner lumen offset from said central longitudinal axis, and cutting flutes on one side of the circumference of the dilator tube, so that when the dilator tube is rotated slightly back and forth, it is configured to perform a foraminoplasty on a vertebra on the side where the cutting flutes are located while protecting the exiting nerve.

US 2016/296344 A1 describes a bone graft delivery system including an elongate tube and a handle having a trigger and a ratcheting mechanism and US 2007/010822 A1 is directed to a bone preparation device for preparing a bone surface for receiving an implant.

In view of the above, there is a need for an improved surgical reamer for removing bone which allows for sensitive areas to be protected during the bone removal process.

### Summary of the Invention

Accordingly, it is an object of the present invention to provide a surgical reamer for removing bone in which protection is afforded to nerves, tissue and/or dura during penetration of the patient in order to facilitate medical treatment besides extremely sensitive areas of an organism or human body so as to accelerate the healing process after a surgery. Further, the present invention aims to provide a set of reamers for removing bone with a surgical reamer which also allows protecting sensitive areas during the removal of bone material.

These goals are achieved by a surgical reamer with the technical features of claim 1 and a set of surgical reamers with the technical features of claim 14. Preferred embodiments of the invention follow from the dependent claims.

According to the invention, there is provided a surgical reamer for removing bone, e.g. to access the spinal canal, the surgical reamer comprising: a shaft extending longitudinally along the length of the surgical reamer; and a cutting section for cutting bone, the cutting section being situated at a distal portion of the shaft, wherein the cutting section is provided at a lateral surface of the shaft so as to extend along part of the circumference of the shaft, and the cutting section extends along less than 50% of the circumference of the shaft.

The terms "distal" and "proximal" are used herein in the sense that is usually assigned to them in connection with surgical instruments, namely the term "distal" indicating the end of a surgical instrument remote from the surgeon when the instrument is in use and the term "proximal" indicating the end of the surgical instrument that is near to a surgeon when the surgical instrument is in use. In other words, the distal end is the end of the surgical instrument which is inserted first into a patient's body and which typically is the end for manipulating the respective surgery side and the proximal end being in the hands of the surgeon.

The term "reamer" is used herein in the sense that it refers to a surgical tool being able to cut sideways, i.e., in lateral directions, by means of a cutting section provided at a lateral surface of a shaft of the reamer. That means the reamer cuts on the "side" and the tip of such a reamer has, unlike a drill, no front-cutting capability.

The "circumference" of the surgical reamer at a distal end of the shaft consists of the cutting section, which extends over less than 50% of the circumference, and the remaining non-cutting section, which extends over the remainder of the circumference of the shaft (over more than 50% of the circumference of the shaft), in order to achieve a closed curve as seen in a cross-section perpendicular to a longitudinal direction of the shaft.

In other words, the "non-cutting section" is a protective surface for protecting sensitive areas in the body during the removal process. That means the protective surface is the non-cutting section on the lateral surface ("circumference") of the reamer which is not part of the cutting section and extends over more than 50% of the shaft, as the cutting section spreads around less than 50% of the circumference of the shaft. Preferably, the protective surface is smooth and/or flat. That means it has a small degree of surface roughness with a maximum upper level of 200µm, preferably less than 100µm, more preferably less than 50µm and even more preferably less than 25µm, so that harm to the sensitive areas can be avoided and the insertion of the reamer is facilitated.

The "cutting section" is a continuous cutting section without gaps in a circumferential direction and the cutting section does not extend over more than 50% of the circumference, so that a selective cutting by oscillating the reamer is achievable. As the "non-cutting" section is also a continuous section spreading over the remaining circumference section of the shaft, it is at the same time possible to protect the tissue, nerves, etc. (sensitive areas) during the cutting process. Thus, it is furthermore conceivable to achieve a sufficient space for cutting bone material without harming the surrounding tissue, nerves, etc.

Therefore, the reamer allows bone material to be removed while protecting surrounding sensitive areas inside the patient.

In some embodiments, the surgical reamer further comprises a non-cutting protective tip for protecting nerves, tissue and/or dura from being damaged, wherein the protective tip extends distally of a distal end of the shaft.

The "protective tip", as defined above, extends along the whole circumference of the shaft and extends from a distal end of the shaft. Such a configuration allows for the area arranged distally of the tip of the cutting section in the body of a patient to be particularly reliably protected. Especially, as the protective tip is arranged at the distal end of the reamer and as the reamer has no front cutting capability, the cutting itself can take place on the circumference of the reamer and the surrounding tissue at the distal end of the reamer is protected from being harmed by the reamer.

According to another aspect of the invention, a cylindrical portion between the proximal end of the protective tip and the distal end of the shaft is provided.

Such a configuration allows an easier, cheaper and more precise manufacturing of the reamer.

The non-cutting protective tip may be cone-shaped.

That means, the diameter of the tip decreases from the proximal end to the distal end of the reamer, such that the reamer has a tapered (a conical) protective tip. Such a configuration enables particularly "smooth" insertion of the reamer and/or a particularly reliable guidance of the tool during the insertion process. That means, a conical protective tip of the reamer allows inserting the reamer without having a perfectly straight hole or a slightly reduced diameter in the body of the patient. Additionally the guiding along a pre-formed hole to the region to be treated can be particularly reliably achieved. With such a configuration, there is thus provided a simple and reliable method for guiding the surgical reamer towards the area, where the cutting should be executed.

The surgical reamer may be a surgical reamer for removing bone to access the spinal canal.

The reamer of the invention can be employed particularly advantageously for such applications since the spinal canal is an especially sensitive area with many parts that need protection and the reamer can protect these parts with its protective area and the oscillating movement of the reamer.

In some embodiments, the surgical reamer is configured to perform an oscillating rotational movement along the circumferential direction of the shaft.

With such a configuration, the cutting can be achieved by an oscillating rotation along the circumferential axis of the shaft. That means the reamer is rotated around an appropriately chosen amount of degrees, so that the nerves, tissue, etc., which are surrounding the bone material in the human body of the reamer are not damaged. The chosen amount of rotational degrees can be calculated by and earlier analysis of the surrounding tissue in the patient. Thus, tissue and nerves surrounding the area in which the removal of material should be achieved can be protected in a particularly reliable manner, as the cutting section only extends over less than 50% of the circumference of the shaft and the remaining area is a protective surface on the shaft. As the reamer is only rotated by part of a full turn and as the reamer has a protective surface, the surrounding area of the bone material to be cut may be protected because the cutting section is never in contact with the surrounding area.

The present invention also provides a surgical system comprising the reamer of the invention and a control, wherein the control is configured to control movement of the reamer.

The control may be configured to control the reamer, such that the reamer performs an oscillating rotational movement along the circumferential direction of the shaft.

With such an oscillating rotational movement along a certain percentage of the circumference of the shaft it can be avoided that the sensitive areas gets in contact with the sharp cutting section during the bone material removal and the sensitive area is thus protected during the operation.

With such a surgical system comprising the reamer of the invention and a control, a particularly precise control of the oscillation parameters may be achievable during the bone removal process inside the human body, i.e. frequency of the oscillation, insertion depth, and the force being applied to the cutting section on the shaft. With such a system, further information regarding the bone structure and the arising forces on the surgical reamer may be generated, so that it is feasible to adapt the parameters of the control of the reamer according to the situation in the body.

This control allows adjusting the parameter of the removal of bone material by an oscillating rotational movement of the surgical reamer during the bone removal.

This leads to a reduced risk of inflammation and allows a faster healing of the excoriation.

The control may be configured to control movement of the reamer so as to remove bone

The control may be configured to control movement of the reamer so that the shaft of the reamer is driven by the control that rotates the reamer clockwise and anti-clockwise along a circumferential direction of the shaft and thereby the cutting section is rotated by a certain percentage of the circumference and can therefore be in contact with the bone material of the human body.

In a preferred embodiment, the cutting section extends along less than 45% of the circumference of the surgical reamer, preferably between 25% and 45%, particularly preferably 35%.

Therefore a precise removal of bone material can be achieved by oscillating the reamer around a longitudinal axis in an appropriate percentage of the circumference in order to protect the surrounding tissue and nerves. That means, with a cutting section extending along less than 45% of the circumference of the surgical reamer, it is conceivable to rotate the surgical reamer by a larger percentage of the circumference of the surgical reamer along the rotational direction of the shaft. This allows the desired portion of bone material in the cutting section to be removed and keeps the opposite side of the cutting section protected, so as to particularly reliably avoid harming the body of the patient while removing the bone material.

In an embodiment, the cutting section extends along at least 10%, preferably at least 20%, more preferably at least 30% of the circumference of the surgical reamer.

With such a configuration, an appropriate amount of bone removal can be achieved and therefore the operation duration can be reduced. Additionally, the process of removing bone can be accelerated, while the nerve and tissue material can be protected from being harmed. Furthermore, having a cutting section in at least 10% of the circumference of the shaft allows a precise removal of bone material. That means, when a small circumferential percentage of the distal end of the shaft is covered with the cutting section and the remaining circumferential section is covered with the continuous protective surface, it is achievable to particularly reliably avoid harming the body of the patient while removing the bone material.

In a preferred embodiment, the cutting section extends in the longitudinal direction of the shaft over at least 10mm, more preferably at least 12 mm, preferably between 15mm and 50mm.

With such a configuration, the cutting section is sized particularly appropriately for cutting into a spinal bone while protecting the surrounding area.

According to the invention, the cutting section has the shape of a segment of a circle in a cross-section perpendicular to the longitudinal direction of the shaft.

According to the invention, the cutting section comprises a plurality of blade members, each blade member substantially extending along the longitudinal direction of the shaft.

Thus, splitting up the applied cutting forces over a plurality of blade members may be achievable, so that the forces being applied to the patient's body and the cutting area inside the body of the patient can be reduced. This leads to a reduced risk of damage inside the human body and allows an easier operation of the patient. Also the risk of a stuck reamer can be avoided. That means, bone material with an inconsistent density can be cut properly and it can be avoided that one single blade member gets stuck inside the bone material.

In an embodiment, the cutting section comprises sharp spots and/or turbine blades and/or sharp edges and/or cutting flutes and/or straight blades.

With such a configuration, adapting the cutting section to the corresponding operational conditions and the surrounding area may be conceivable. That means, that the amount of injuries or risks of harming the human body can be reduced and the cutting section can be adapted to the current situation inside the patient's body.

According to the invention, the shaft comprises a guide channel extending in the longitudinal direction of the shaft such that a guide wire can be inserted into the guide channel through the shaft.

With such a configuration, particularly reliably centring the position of the bone where the cutting is to be performed may be achievable. That means a guide wire can be inserted into the human body before the surgical reamer is inserted. By doing so, avoiding harming surrounding veins, arteries, and nerves by guiding the surgical reamer along the guide wire through the guide channel being in the centre of the longitudinal direction of the shaft may be conceivable. Additionally, it is possible to avoid a stimulation of nerves, tissue and/or it is possible to achieve that the surgical reamer and its cutting section can be oriented away from the tissue to be protected. With such a configuration, the reamer can receive a guide wire in order to improve the precision of the positioning of the cutting section for more precise reaming.

In an embodiment, the shaft has an outer diameter in the range of 2.0mm to 10.0mm.

With such a configuration, the cutting section is sized particularly appropriate for cutting into a spinal bone.

In a specific embodiment, the diameter of the protective tip at a proximal end of the protective tip is substantially the same as the diameter of the shaft.

With such a configuration, no further edges around the circumference of the reamer are existent between the protective tip and the distal end of the shaft and an influence of an edge in a circumferential direction between the protective tip and the cutting section can be avoided. This reduces the risk of an injury during the removal of bone material.

In some embodiments, the diameter of the protective tip at a proximal end of the protective tip is smaller than the diameter of the shaft, preferably by more than 0.1mm, more preferably by more than 0.5mm, even more preferably by 1 mm.

In such a configuration, there is a gap between the protective tip and the inner part of the cutting section and/or the shaft in a radial direction, so that the protective tip is not influencing the removal of bone material and the protective tip helps for a first insertion into the patient's body.

According to another embodiment, the protective tip is positioned radially inside the cutting section.

With such a configuration, the reaming area may be maximized for the cross sectional area of the surgical reamer while still providing a protective tip for an easier insertion of the surgical reamer.

The invention further provides a set of surgical reamers which comprises at least a first surgical reamer and a second surgical reamer according to any one of the preceding aspects, wherein the diameter of the shaft of the second surgical reamer is larger than the diameter of the shaft of the first surgical reamer.

With such a set of reamers a stepwise opening of a hole in a bone material can be achieved by extending the width of the hole by starting from a first reamer having a small diameter and enhancing the diameter of the reamer step by step to the second reamer. Further, the diameter of the hole in the bone material can be extended by further reamers having even larger diameters than the second reamer.

In some embodiments, the surgical reamers used in the set of reamers may differ from each other in the shape of their cutting faces.

Also disclosed herein is a method for removing bone with a surgical reamer according to any of the above-mentioned aspects. The method for removing bone with a surgical reamer comprises the following steps: positioning the surgical reamer at the location at which bone material is to be removed; and performing an oscillating rotational movement of the surgical reamer along the circumferential direction of the shaft, wherein the rotational movement of the surgical reamer oscillates between rotational movement in the clockwise direction and rotational movement in the anticlockwise direction, and in each of the rotational movement in the clockwise direction and the rotational movement in the anticlockwise direction, the surgical reamer rotates by only part of a full turn of the surgical reamer, preferably by 80% or less of a full turn.

As the continuous cutting section of the reamer extends along less than 50% of the circumference of the reamer, and the remaining circumferential section (which is the non-cutting protective surface at a distal end of the shaft) extends over more than 50% of the circumference of the reamer, the reamer is sized particularly appropriately for protecting the surrounding sensitive area while bone material is removed in an above disclosed manner.

By the oscillating rotational movement of the surgical reamer, a contact between the bone material and the cutting section of the surgical reamer without a rotational movement by a full circle may be achieved. By doing so, the reaming process is started and bone material is removed by every oscillating rotational movement along the circumference of the shaft in the clockwise and anticlockwise direction. By moving only a part of a full turn (preferably less than 80% of a full turn, particularly preferably less than 50% of a full turn), the opposing sensitive area (such as tissue or nerves) of a patient's body can be protected, as the cutting section of the reamer is not contacting the sensitive area. Thereby, serious damage and injuries inside the human body can be avoided and the healing process after the surgery can be accelerated.

After the removal of bone material with the surgical reamer is finished, another surgical reamer having a shaft with a larger diameter can be positioned at the location at which bone material is to be removed.

With such a configuration, a widening of a hole inside a bone material while keeping the injuries and harming to the surrounding tissue as low as possible may be achieved. Additionally, a quicker healing may be achieved.

Furthermore, the surgical reamer can be guided to the location at which bone material is to be removed by means of a guide wire, wherein the guide wire is inserted in a guide channel extending in the longitudinal direction of the shaft of the surgical reamer.

With such a configuration, improving the precision of the positioning of the surgical reamer for more precise reaming may be achievable. Additionally, harming the material surrounding the cutting area with the cutting section can be avoided and inserting different reamers can be achieved easily. By doing so, widening of a hole inside the bone material is facilitated and an operation process is accelerated by speeding up the positioning step. By doing so, in general, the operation can be improved, the risk of harming the patient can be reduced, and the healing process is accelerated.

The blades of the cutting section may extend substantially parallel to the longitudinal axis of the shaft.

With such a configuration, reduced manufacturing costs and a facilitated manufacturing process may be achieved.

The blades of the cutting section may be (slightly) tilted relative to the longitudinal axis of the shaft, e.g., having an angle of rifling within a range of 1° to 30°, preferably within a range of 1° to 15°, more preferably within a range of 1° to 8° and even more preferably within a range of 1° to 4° to the longitudinal axis of the shaft.

That leads to the technical effect of an improved bone removal, as the removed bone material can be transported easily through the tilted blades.

### Brief Description of the Drawings

For a better understanding of the present invention and to see how the same may be carried into effect, reference will now be made, by way of example only, to the accompanying drawings, in which:
Figure 1 shows a side view of a surgical reamer according to an exemplary embodiment of the present invention;
Figure 2 shows a cross sectional view of the surgical reamer according to the exemplary embodiment, taken along lines A-A in Figure 1;
Figure 3 shows an enlarged view of the distal end of the surgical reamer according to the exemplary embodiment of the present invention, as indicated by the encircled area Z in Figure 1; and
Figure 4 shows a bottom view of the surgical reamer according to the exemplary embodiment of the present invention taken from the distal end of the reamer.

### Detailed Description of Preferred Embodiments

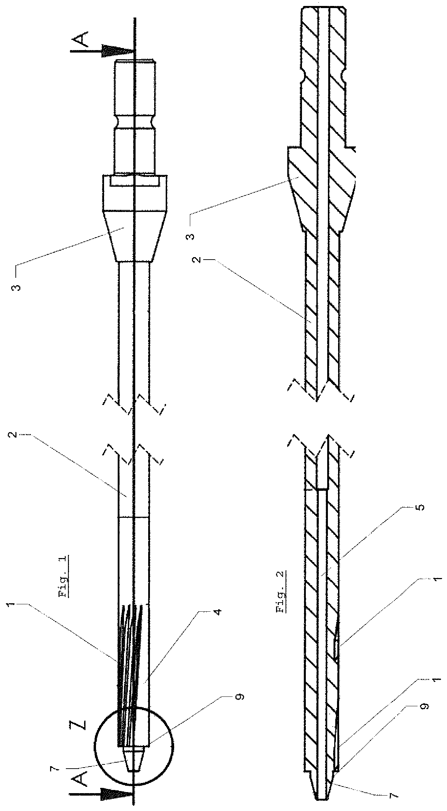

Figures 1 and 2 show a surgical reamer having a continuous cutting section 1 for cutting bone inside a patient's body, wherein the cutting section 1 is disposed at a distal portion of a shaft 2 shown in Figure 1. Besides the cutting section 1, the surgical reamer also has a continuous non-cutting section 4 in the remaining circumference of the distal end of the shaft for protecting the surrounding tissue during the removal of bone material. Additionally, the reamer has a protective tip 7 being arranged at a distal portion of the shaft. Further, the shaft has a guide channel which extends along the center of the longitudinal axis of the shaft. Additionally, the surgical reamer of the exemplary embodiment also has a connector 3, for connecting the surgical reamer to a driving system and/or a control, or the like.

As can be taken from Figure 1, the cutting section 1 extends along less than 50% (in this exemplary embodiment approximately 25%) of the circumference of the shaft 2 and is arranged at a lateral surface of the shaft 2. The part of the circumference of the shaft 2 which is not covered by the cutting section 1 is covered by the non-cutting section 4, also arranged at the distal end of the shaft 2 of the surgical reamer. This non-cutting section 4 is arranged to protect the tissue in the patient which should be protected from being injured or harmed by the sharp cutting section. Therefore, the surface of the non-cutting section 4 should be designed smooth and even.

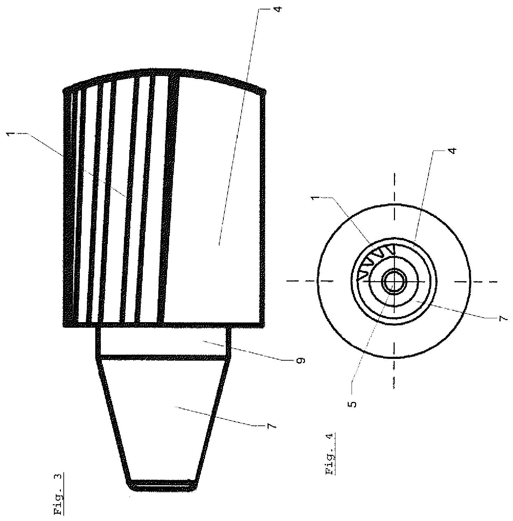

As can be seen from Figure 3, the cutting section 2 comprises four blades in the cutting section 1 spread around the cutting section with a constant sequence, the blades being configured to cut when the reamer is oscillated in clockwise and anti-clockwise directions over a certain percentage of a full turn. This percentage is dependent on the conditions inside the patient's body, i.e. the surrounding nerves or tissue.

As is shown in Figure 4, the four sharp blades have an angle of rifling of 3° to the longitudinal axis, which can be used for cutting and removing bone by reaming along the circumference (the side) of the reamer.

The non-cutting protective tip 7 extends distally of a distal end of the shaft 2. In this orientation, the protective tip 7 provides protection for the tissue, nerves and/or dura of the patient by extending distally of the distal end of the cutting section. As can be taken from Figures 1 and 2, in this exemplary embodiment, the protective tip 7 shows a smaller diameter than the shaft 2. In other embodiments, it may also be feasible that the proximal end of the protective tip 7 has the same diameter as the distal end of the shaft, so that no edges are existent at this position of the shaft. Other shapes of the protective tip 7, such as cylindrical or the like, are also possible without any further limitation.

As can be taken from Figures 1 and 2, the reamer has a cylindrical portion 9 which is arranged between the proximal end of the protective tip 7 and the distal end of the shaft 2 having a smaller diameter than the shaft 2 and the same diameter as the proximal end of the protective tip.

In general, the surgical reamer can be driven by a control (not shown) for controlling the reamer and a driving mechanism (not shown) for driving the reamer. The control is connected to the surgical reamer by the connector 3. This connector 3 can have a standardized shape or connection standard which allows transferring forces in a circumferential direction. Further, the control and the driving mechanism may allow a movement in a radial or axial movement of the reamer as well.

Most importantly, the connector 3 is used to transmit rotational forces so as to achieve an oscillating movement of the shaft 2. Thus, removing the bone material by an oscillating movement of the shaft 2 is achievable. That means, the reamer is not moved around the whole circumference, but moves clockwise and anticlockwise by a certain frequency to remove bone material.

In Figure 1, the guide channel 5 of the surgical reamer is also shown. The guide channel 5 is a through hole along the center of the longitudinal axis of the shaft for receiving a guide wire (not shown), which is guided through the guide channel. The guide wire can be a thin wire which allows facilitating insertion into the patient's body.

Figures 1 to 4 all show the same reamer of an exemplary embodiment of the invention. Additionally, it is also feasible to use the described reamer in a set of reamers which differ from each other in at least one parameter. That means all reamers have the same general configuration of the invention but differ in one parameter. For example, a set of reamers according to the invention can comprise a plurality of reamers with each reamer of the set of reamers having a different diameter. Furthermore, for example, the set of reamers may also comprise a plurality of reamers having different cutting sections, different blades, or a different amount of blades in the continuous cutting section.

Apart from the aforementioned exemplary embodiments, which teach the manner in which the invention may be carried into effect, further alternative embodiments are conceivable. In particular, there are a number of variations which are possible, as may be appreciated by those skilled in the art.

Now, a method for removing bone with a surgical reamer, as described above, is described. First of all, after opening the patient's body, the surgical reamer is guided towards the area, where bone material is to be removed. In an exemplary embodiment of the method, guiding the surgical reamer by a guide wire running through the guide channel 5 of the surgical reamer may be feasible. By this guide wire, it is also achievable to optically check the situation and the surrounding of the area to be cut with an optical system being inserted before the beginning of the insertion of the surgical reamer.

After the situation inside the patient is checked and the position of the material to be removed and the surrounding tissue and, for example, the position of nerves which should be protected is detected, the surgical reamer can be positioned at the starting position inside the human body.

In a next step, an oscillating rotational movement of the surgical reamer along the circumferential direction of the shaft is started. Thereby, the force is transmitted via a control and a corresponding driving mechanism (not shown). Thereby, the rotational movement of the surgical reamer oscillates between a rotational movement in the clockwise and a rotational movement in the anticlockwise direction. Thereby, the cutting section 1 of the shaft 2 is in contact with the bone material to be removed and the non-cutting section 4 meanwhile protects the nerves and surrounding tissue from being harmed through the cutting section 1. Therefore, the percentage of a circumferential movement could be determined accordingly during the optical check before the removal starts. Therefore, the rotational movement in the clockwise and the anticlockwise direction may be only a part of a full turn of the surgical reamer, preferably 80% or less of a full turn. By doing so, the tissue on the opposite side of the cutting section is protected.

According to another step of an exemplary embodiment of the method for removing bone by a surgical reamer, replacing the currently used surgical reamer by another reamer having a larger diameter may be possible. Therefore the first surgical reamer is pulled out of the patient's body and the second surgical reamer having a larger diameter is guided to the current position by the guide wire passing through the guide channel 5. Thereby, the forces for rotating the reamer having a larger diameter are also transmitted by the standardized connector 3 and the corresponding control and drive mechanism. Therefore, the width of the hole, in which the bone material should be removed, is widened.

Guiding the surgical reamer without a guide wire or the like may be also be practicable.

In fact, the 25% circumferential cutting section allows a particularly appropriate cutting of bone material while the surrounding area is protected. In case of having sensitive tissue arranged at the opposite side of the cutting section, it is attainable to move the reamer around a sizeable oscillating rotation around another 25% of the circumference without ever contacting the sensitive area with the cutting section.

In light of this, there will be many alternatives which implement the teaching of the present invention. It is expected that one skilled in the art will be able to modify and adapt the above disclosure to suite its own circumstances and requirements within the scope of the present invention, which is defined by the appended claims.

## Claims

1. A surgical reamer for removing bone, the surgical reamer comprising:
a shaft (2) extending longitudinally along the length of the surgical reamer; and
a cutting section (1) for cutting bone, the cutting section (1) being situated at a distal portion of the shaft (2), wherein
the cutting section (1) is provided at a lateral surface of the shaft (2) so as to extend along part of the circumference of the shaft (2), and
the cutting section (1) extends along less than 50% of the circumference of the shaft (2),
in a cross-section perpendicular to the longitudinal direction of the shaft (2), the cutting section (1) has the shape of a segment of a circle, and
the cutting section (1) comprises a plurality of blade members, each blade member substantially extending along the longitudinal direction of the shaft (2),
the shaft (2) comprises a guide channel (5) extending in the longitudinal direction of the shaft (2), such that a guide wire can be inserted into the guide channel (5) through the shaft (2).
**characterized in that**
the guide channel (5) extends along the center of the longitudinal axis of the shaft (2).

2. The surgical reamer according to claim 1, further comprising a non-cutting protective tip (7) for protecting nerves, tissue and/or dura from being damaged, wherein the protective tip (7) extends distally of a distal end of the shaft (2).

3. The surgical reamer according to claim 2, wherein the non-cutting protective tip (7) is cone-shaped.

4. The surgical reamer according to any one of the preceding claims, wherein the surgical reamer is for removing bone to access the spinal canal.

5. The surgical reamer according to any one of the preceding claims, wherein the surgical reamer is configured to perform an oscillating rotational movement along the circumferential direction of the shaft (2).

6. The surgical reamer according to any one of the preceding claims, wherein the cutting section (1) extends along less than 45% of the circumference of the surgical reamer, preferably between 25% and 45%, particularly preferably 35%.

7. The surgical reamer according to any one of the preceding claims, wherein the cutting section (1) extends along at least 10%, preferably at least 20%, of the circumference of the surgical reamer.

8. The surgical reamer according to any one of the preceding claims, wherein the cutting section (1) extends in the longitudinal direction of the shaft (2) over at least 10mm, preferably between 15mm and 50mm.

9. The surgical reamer according to any one of the preceding claims, wherein the cutting section (1) comprises sharp spots and/or turbine blades and/or sharp edges and/or cutting flutes and/or straight blades.

10. The surgical reamer according to any one of the preceding claims, wherein the shaft (2) has an outer diameter in the range of 2.0mm to 10.0mm.

11. The surgical reamer according to claim 2 or any one of claims 3 to 10 as dependent on claim 2, wherein the diameter of the protective tip (7) at a proximal end of the protective tip is substantially the same as the diameter of the shaft.

12. The surgical reamer according to claim 2 or any one of claims 3 to 10 as dependent on claim 2, wherein the diameter of the protective tip (7) at a proximal end of the protective tip is smaller than the diameter of the shaft (2), preferably by more than 0.1mm, more preferably by more than 0.5mm.

13. The surgical reamer according to claim 2 or any one of claims 3 to 10 as dependent on claim 2, wherein the protective tip (7) is positioned radially inside the cutting section (1).

14. A set of surgical reamers comprising at least a first surgical reamer and a second surgical reamer according to any one of the preceding claims, wherein the diameter of the shaft (2) of the second surgical reamer is larger than the diameter of the shaft (2) of the first surgical reamer.

## Patentansprüche

1. Chirurgischer Reamer zur Entfernung von Knochen, wobei der chirurgische Reamer Folgendes umfasst:
einen Schaft (2), der sich in Längsrichtung entlang der Länge des chirurgischen Reamers erstreckt; und
einen Schneidabschnitt (1) zum Schneiden von Knochen, wobei sich der Schneidabschnitt (1) an einem distalen Abschnitt des Schafts (2) befindet, wobei
der Schneidabschnitt (1) an einer Mantelfläche des Schafts (2) vorgesehen ist, so dass er sich entlang eines Teils des Umfangs des Schafts (2) erstreckt, und
der Schneidabschnitt (1) sich entlang weniger als 50 % des Umfangs des Schafts (2) erstreckt,
der Schneidabschnitt (1) in einem Querschnitt senkrecht zur Längsrichtung des Schafts (2) die Form eines Kreissegments aufweist, und
der Schneidabschnitt (1) eine Vielzahl von Klingenelementen umfasst, wobei sich jedes Klingenelement im Wesentlichen entlang der Längsrichtung des Schafts (2) erstreckt,
der Schaft (2) einen Führungskanal (5) umfasst, der sich in der Längsrichtung des Schafts (2) erstreckt, so dass ein Führungsdraht durch den Schaft (2) in den Führungskanal (5) eingeführt werden kann.
**dadurch gekennzeichnet, dass**
der Führungskanal (5) sich entlang der Mitte der Längsachse des Schafts (2) erstreckt.

2. Chirurgischer Reamer nach Anspruch 1, der weiter eine nicht schneidende Schutzspitze (7) zum Schützen von Nerven, Gewebe und/oder Dura vor Beschädigung umfasst, wobei sich die Schutzspitze (7) distal eines distalen Endes des Schafts (2) erstreckt.

3. Chirurgischer Reamer nach Anspruch 2, wobei die nicht schneidende Schutzspitze (7) kegelförmig ist.

4. Chirurgischer Reamer nach einem der vorstehenden Ansprüche, wobei der chirurgische Reamer zur Entfernung von Knochen vorgesehen ist, um Zugang zum Spinalkanal zu erhalten.

5. Chirurgischer Reamer nach einem der vorstehenden Ansprüche, wobei der chirurgische Reamer ausgebildet ist, eine oszillierende Rotationsbewegung in der Umfangsrichtung des Schafts (2) auszuführen.

6. Chirurgischer Reamer nach einem der vorstehenden Ansprüche, wobei sich der Schneidabschnitt (1) entlang weniger als 45 % des Umfangs des chirurgischen Reamers , bevorzugt zwischen 25 % und 45 %, besonders bevorzugt 35 % erstreckt.

7. Chirurgischer Reamer nach einem der vorstehenden Ansprüche, wobei sich der Schneidabschnitt (1) entlang mindestens 10 %, bevorzugt mindestens 20 %, des Umfangs des chirurgischen Reamers erstreckt.

8. Chirurgischer Reamer nach einem der vorstehenden Ansprüche, wobei sich der Schneidabschnitt (1) in der Längsrichtung des Schafts (2) über mindestens 10 mm, bevorzugt zwischen 15 mm und 50 mm, erstreckt.

9. Chirurgischer Reamer nach einem der vorstehenden Ansprüche, wobei der Schneidabschnitt (1) scharfe Stellen und/oder Turbinenschaufeln und/oder scharfe Kanten und/oder Schneidnuten und/oder gerade Klingen umfasst.

10. Chirurgischer Reamer nach einem der vorstehenden Ansprüche, wobei der Schaft (2) einen Außendurchmesser im Bereich von 2,0 mm bis 10,0 mm aufweist.

11. Chirurgischer Reamer nach Anspruch 2 oder einem der Ansprüche 3 bis 10 in Abhängigkeit von Anspruch 2, wobei der Durchmesser der Schutzspitze (7) an einem proximalen Ende der Schutzspitze im Wesentlichen dem Durchmesser des Schafts entspricht.

12. Chirurgischer Reamer nach Anspruch 2 oder einem der Ansprüche 3 bis 10 in Abhängigkeit von Anspruch 2, wobei der Durchmesser der Schutzspitze (7) an einem proximalen Ende der Schutzspitze kleiner ist als der Durchmesser des Schafts (2), bevorzugt um mehr als 0,1 mm, mehr bevorzugt um mehr als 0,5 mm.

13. Chirurgischer Reamer nach Anspruch 2 oder einem der Ansprüche 3 bis 10 in Abhängigkeit von Anspruch 2, wobei die Schutzspitze (7) radial innerhalb des Schneidabschnitts (1) positioniert ist.

14. Satz chirurgischer Reamer, der mindestens einen ersten chirurgischen Reamer und einen zweiten chirurgischen Reamer nach einem der vorstehenden Ansprüche umfasst, wobei der Durchmesser des Schafts (2) des zweiten chirurgischen Reamers größer ist als der Durchmesser des Schafts (2) des ersten chirurgischen Reamers.

## Revendications

1. Alésoir chirurgical destiné à enlever de l'os, l'alésoir chirurgical comprenant :
une tige (2) s'étendant longitudinalement sur la longueur de l'alésoir chirurgical ; et
une section de coupe (1) destinée à couper de l'os, la section de coupe (1) étant située au niveau d'une partie distale de la tige (2), dans lequel
la section de coupe (1) est disposée au niveau d'une surface latérale de la tige (2) de manière à s'étendre le long d'une partie de la circonférence de la tige (2), et
la section de coupe (1) s'étend sur moins de 50 % de la circonférence de la tige (2),
dans une section transversale perpendiculaire à la direction longitudinale de la tige (2), la section de coupe (1) présente la forme d'un segment de cercle, et
la section de coupe (1) comprend une pluralité d'éléments de lame, chaque élément de lame s'étendant sensiblement le long de la direction longitudinale de la tige (2),
la tige (2) comprend un canal de guidage (5) s'étendant dans la direction longitudinale de la tige (2), de sorte qu'un fil-guide peut être inséré dans le canal de guidage (5) à travers la tige (2),
**caractérisé en ce que**
le canal de guidage (5) s'étend le long du centre de l'axe longitudinal de la tige (2).

2. Alésoir chirurgical selon la revendication 1, comprenant en outre une pointe de protection non coupante (7) pour empêcher les nerfs, le tissu et/ou la dure-mère d'être endommagés, dans lequel la pointe de protection (7) s'étend de manière distale par rapport à une extrémité distale de la tige (2).

3. Alésoir chirurgical selon la revendication 2, dans lequel la pointe de protection non coupante (7) est en forme de cône.

4. Alésoir chirurgical selon l'une quelconque des revendications précédentes, dans lequel l'alésoir chirurgical est destiné à enlever de l'os pour accéder au canal rachidien.

5. Alésoir chirurgical selon l'une quelconque des revendications précédentes, dans lequel l'alésoir chirurgical est configuré pour effectuer un mouvement de rotation oscillant le long de la direction circonférentielle de la tige (2).

6. Alésoir chirurgical selon l'une quelconque des revendications précédentes, dans lequel la section de coupe (1) s'étend sur moins de 45 % de la circonférence de l'alésoir chirurgical, de préférence entre 25 % et 45 %, en particulier de préférence sur 35 %.

7. Alésoir chirurgical selon l'une quelconque des revendications précédentes, dans lequel la section de coupe (1) s'étend le long d'au moins 10 %, de préférence d'au moins 20 %, de la circonférence de l'alésoir chirurgical.

8. Alésoir chirurgical selon l'une quelconque des revendications précédentes, dans lequel la section de coupe (1) s'étend dans la direction longitudinale de la tige (2) sur au moins 10 mm, de préférence entre 15 mm et 50 mm.

9. Alésoir chirurgical selon l'une quelconque des revendications précédentes, dans lequel la section de coupe (1) comprend des pointes acérées et/ou des lames d'aube et/ou des arêtes vives et/ou des goujures de coupe et/ou des lames droites.

10. Alésoir chirurgical selon l'une quelconque des revendications précédentes, dans lequel la tige (2) présente un diamètre externe dans la plage de 2,0 mm à 10,0 mm.

11. Alésoir chirurgical selon la revendication 2 ou l'une quelconque des revendications 3 à 10 lorsqu'elle dépend de la revendication 2, dans lequel le diamètre de la pointe de protection (7) au niveau d'une extrémité proximale de la pointe de protection est sensiblement le même que le diamètre de la tige.

12. Alésoir chirurgical selon la revendication 2 ou l'une quelconque des revendications 3 à 10 lorsqu'elle dépend de la revendication 2, dans lequel le diamètre de la pointe de protection (7) au niveau d'une extrémité proximale de la pointe de protection est plus petit que le diamètre de la tige (2), de préférence de plus de 0,1 mm, de manière davantage préférée de plus de 0,5 mm.

13. Alésoir chirurgical selon la revendication 2 ou l'une quelconque des revendications 3 à 10 lorsqu'elle dépend de la revendication 2, dans lequel la pointe de protection (7) est positionnée radialement à l'intérieur de la section de coupe (1).

14. Ensemble d'alésoirs chirurgicaux comprenant au moins un premier alésoir chirurgical et un deuxième alésoir chirurgical selon l'une quelconque des revendications précédentes, dans lequel le diamètre de la tige (2) du deuxième alésoir chirurgical est plus grand que le diamètre de la tige (2) du premier alésoir chirurgical.
